(19) Europäisches Patentamt
European Patent Office
Office européen des brevets

(11) EP 2 119 440 A1

(12) # EUROPEAN PATENT APPLICATION

(43) Date of publication:
**18.11.2009 Bulletin 2009/47**

(21) Application number: **09011337.4**

(22) Date of filing: **17.05.2006**

(51) Int Cl.:
***A61K 31/245*** *(2006.01)*
***A61K 31/4168*** *(2006.01)*
***A61P 21/04*** *(2006.01)*
***A61P 25/00*** *(2006.01)*
***A61P 25/02*** *(2006.01)*
***A61P 25/14*** *(2006.01)*
***A61P 25/16*** *(2006.01)*
***A61P 25/28*** *(2006.01)*
***A61P 27/02*** *(2006.01)*
***A61P 27/06*** *(2006.01)*
***C07D 233/50*** *(2006.01)*

(84) Designated Contracting States:
**AT BE BG CH CY CZ DE DK EE ES FI FR GB GR HU IE IS IT LI LT LU LV MC NL PL PT RO SE SI SK TR**

(30) Priority: **17.05.2005 JP 2005143476**

(62) Document number(s) of the earlier application(s) in accordance with Art. 76 EPC:
**06746499.0 / 1 884 237**

(71) Applicant: **SANTEN PHARMACEUTICAL CO., LTD.**
**Higashiyodogawa-ku,**
**Osaka-shi,**
**Osaka 533-8651 (JP)**

(72) Inventors:
- **Oohashi, Kouji**
**Ikoma-shi**
**Nara 630-0101 (JP)**
- **Doi, Reina**
**Ikoma-shi**
**Nara 630-0101 (JP)**
- **Kageyama, Masaaki**
**Ikoma-shi**
**Nara 630-0101 (JP)**

(74) Representative: **Müller-Boré & Partner**
**Patentanwälte**
**Grafinger Strasse 2**
**81671 München (DE)**

Remarks:
This application was filed on 03-09-2009 as a divisional application to the application mentioned under INID code 62.

(54) **Amidino derivatives for use in the prevention or treatment of retinitis pigmentosa and Leber's disease**

(57) The present invention relates to the use of a compound represented by the following general formula [I] or a salt thereof for manufacturing a medicament for preventing or treating retinitis pigmentosa and Leber's disease:

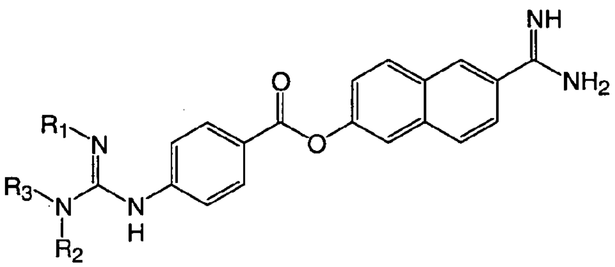

[I]

wherein $R_1$, $R_2$ and $R_3$ are the same or different and represent a hydrogen atom or an alkyl group, or $R_1$ and $R_3$ may be joined together to form a ring having one or more double bonds.

EP 2 119 440 A1

## Description

### Technical Field

The present invention relates to a protective agent for a neuronal cell, containing a compound represented by the general formula [I] or a salt thereof as an active ingredient, and particularly relates to a preventive or therapeutic agent for an eye disease accompanied by optic nerve damage such as a retinal disease or glaucoma.

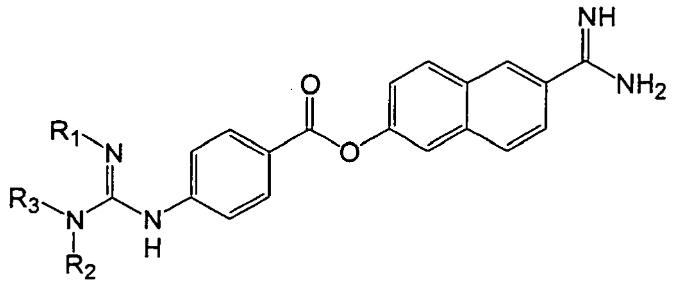

[ I ]

### Background Art

The retina has a function to receive light from outside, and plays an important role for visual function. The retina is structurally a tissue having a thickness of from 0.1 to 0.5 mm which is composed of 10 layers such as retinal pigment epithelium layer, inner plexiform layer, ganglion cell layer and nerve fiber layer. In the inner plexiform layer, there exist neuronal cells called amacrine cells which are paired with ganglion cell neurites to form synapses. Since these neuronal cells respond well both at the start and at the end of light irradiation, they are considered to work as a detector of light intensity. In the ganglion cell layer, there exist ganglion cells which are present in the innermost retina (hereinafter referred to as "RGC") and are deeply involved in motor vision, peripheral vision, color vision, stereoscopic vision and the like. Further, in the nerve fiber layer, retinal vessels which are branches of central artery and vein of retina run and play a role of supplying retinal neuronal cells with oxygen and nutrition.

When retinal vessels are occluded or narrowed because of a cause such as twitching, thrombus or arteriosclerosis, disorder occurs in retinal blood circulation, and thus, the supply of oxygen and nutrition to the retina or optic nerve is blocked. The disorder of retinal blood circulation occupies especially an important position in retinal diseases. Examples of representative symptoms which are accompanied by the disorder of retinal blood circulation include retinal vascular occlusion in which retinal vein or retinal artery is occluded or narrowed, diabetic retinopathy which is one of the causes of retinal detachment, and ischemic optic neuropathy in which visual dysfunction occurs. Further, it is considered that the ganglion cell death is deeply involved also in the incidence of macular degeneration, retinitis pigmentosa, Leber's disease or the like.

Further, glaucoma is one of the eye diseases causing serious visual dysfunction which leads to visual loss if it is not appropriately treated. In glaucoma, among retinal neuronal cells, particularly RGC is selectively damaged and optic nerve damage is caused, which results in progressing to the visual field defect. Therefore, there has now been being established so-called "Neuroprotection", i.e., an idea that a treatment for preventing or minimizing RGC damage will lead to an ultimate treatment of glaucoma (Ophthalmology, 40, 251-273, 1998).

The detailed mechanism of glaucomatous optic nerve damage has not been elucidated yet. However, a mechanical disorder theory, in which optic nerve atrophy is caused by the direct compression of the optic nerve due to an increase in the intraocular pressure, and a circulatory disorder theory, in which a circulatory disorder in the optic papilla is the main cause of optic nerve atrophy, have been proposed, and it is considered that both of these mechanisms based on a mechanical disorder and a circulatory disorder are related in a complex manner. Further, both the mechanical disorder and the circulatory disorder cause an optic nerve axonal transport disorder, and it is considered that the disruption of supply of a neurotrophic factor accompanying this axonal transport disorder is one of the causes of RGC damage (Ophthalmology, 44, 1413-1416, 2002). In addition, glutamate is one of the neurotransmitters in the retina and it is considered that the excess activation of the glutamate signaling cascade by any cause is also one of the causes of RGC damage (Surv. Ophthalmol., 48, S38-S46, 2003).

Accordingly, if there is a drug that has a protective effect on retinal neuronal cells such as RGC, it is expected to be useful for the prevention or treatment of a retinal disease typified by retinal vascular occlusion, ischemic optic neuropathy, retinitis pigmentosa and Leber's disease or an eye disease accompanied by optic nerve damage such as glaucoma.

On the other hand, in JP-A-57-053454 and JP-A-61-33173, it is described that a compound represented by

the general formula [I] or a salt thereof has a trypsin inhibitory activity and is effective in the treatment of pancreatitis. However, it has not been studied at all that what pharmacological action is exhibited by the compound with regard to an eye disease such as a retinal disease or glaucoma.

Disclosure of the invention

Problems to be Solved

It is a very interesting subject to search a new medicinal use of a compound represented by the general formula [I] or a salt thereof.

Means of Solving Problems

The present inventors found that a compound represented by the general formula [I] or a salt thereof exhibits an excellent inhibitory effect on cell death in rat models of retinal damage induced by N-methyl-D-aspartate (hereinafter referred to as "NMDA"), which is an activator for NMDA receptor, being one of the glutamate receptors, and an excitatory amino acid for retinal neuronal cells, and thus the present invention has been accomplished.

That is, the present invention is directed to a protective agent for a retinal neuronal cell such as RGC, comprising as an active ingredient, a compound represented by the general formula [I] or a salt thereof (hereinafter, these are also collectively referred to as "the present compound"), and such a compound is useful as a preventive or therapeutic agent for a retinal disease typified by retinal vascular occlusion, diabetic retinopathy, ischemic optic neuropathy, macular degeneration, retinitis pigmentosa and Leber's disease or an eye disease accompanied by optic nerve damage such as glaucoma. Further, because the present compound has an effect of protecting a neuronal cell, it is also useful as a preventive or therapeutic agent for a central nervous system disorder such as senile dementia, Alzheimer's disease, Parkinson's disease, Huntington's disease, ALS, spinal cord injury, multiple sclerosis, neurodegeneration, muscular atrophy, motor neuron injury, acute central nervous system injury, muscular dystrophy or prion disease.

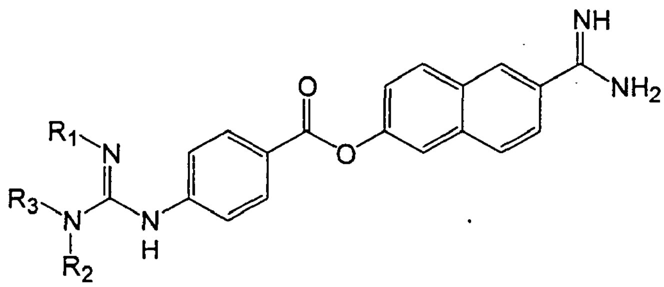

[ I ]

(In the formula, $R_1$, $R_2$ and $R_3$ are the same or different and represent a hydrogen atom or an alkyl group, or $R_1$ and $R_3$ may be joined together to form a ring having one or more double bonds).

Preferred specific examples of the present compound include 6'-amidino-2'-naphthyl-4-guanidinobenzoate dimethanesulfonate represented by the following formula [Ia] (hereinafter referred to as "nafamostat") and 6'-amidino-2'-naphthyl-(4-(4,5-dihydro-1H-2-imidazolyl)amino)benzoate dimethanesulfonate represented by the following formula [Ib] (hereinafter referred to as "FUT-187").

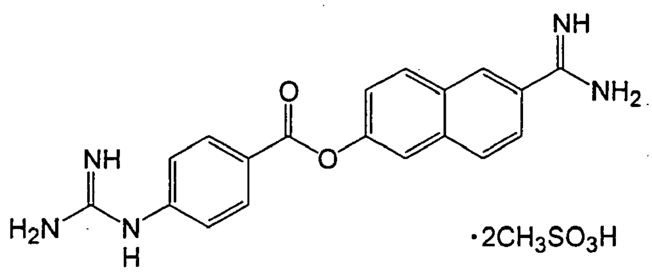

[ I a]

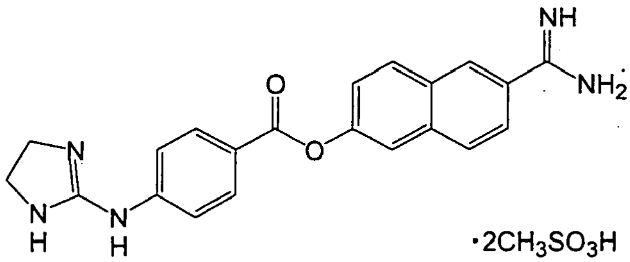

[ I b]

$\cdot 2CH_3SO_3H$

The present compound can be produced in accordance with a method commonly used in the field of organic synthetic chemistry, and the detailed production method is described in JP-A-61-33173.

Hereinafter, the respective groups and words and phrases defined herein will be described. The "alkyl" refers to straight-chain or branched alkyl having 1 to 6 carbon atoms, and specific examples thereof include methyl, ethyl, n-propyl, n-butyl, n-pentyl, n-hexyl, isopropyl, isobutyl, sec-butyl, tert-butyl, isopentyl and the like. Further, the phrase "to form a ring having one or more double bonds" means to form a ring such as 2-imidazolyl, 4,5-dihydro-1H-2-imidazolyl, 2-pyrimidyl, or 1,4,5,6-tetrahydro-2-pyrimidyl.

The present compound can be in the form of a "prodrug". The "prodrug" as stated herein means a compound obtained by modifying the present compound with a group which can leave the compound in vivo, and particular examples thereof include a prodrug in the form of a derivative obtained by acylation or carbamation of a group containing a nitrogen atom which can be acylated such as an amino group or a guanidino group.

The salt of the present compound may be a pharmaceutically acceptable organic or inorganic acid addition salt. Examples of the acid include an inorganic acid such as hydrochloric acid, hydrobromic acid, hydroiodic acid, sulfuric acid, acetic acid or phosphoric acid, and an organic acid such as lactic acid, citric acid, methanesulfonic acid, fumaric acid, maleic acid, mandelic acid, tartaric acid or salicylic acid.

In the present compound, optical isomers exist, and sometimes diastereomers exist, and those containing such an isomer as an active ingredient are also included in the present invention. Further, the present compound can be in the form of a solvate such as a hydrate.

The effect of protecting a retinal neuronal cell of the present compound is based on the results that a compound represented by the above general formula [I] or a salt thereof exhibits an excellent inhibitory effect on retinal neuronal cell death in a pharmacological test for NMDA-induced retinal damage in rats described below.

The present compound can be formulated into a single preparation or a mixed preparation by adding a pharmaceutically acceptable additive as needed using a technique which is commonly used.

When the present compound is used for the prevention or treatment of these eye diseases, it can be administered to a patient orally or parenterally. Examples of the administration method include oral administration, topical administration to eyes (such as instillation administration, intravitreal administration, subconjunctival administration and sub-Tenon's administration), intravenous administration, transdermal administration and the like. Further, it is formulated into a dosage form suitable for administration together with a pharmaceutically acceptable additive as needed. Examples of the dosage form suitable for oral administration include tablets, capsules, granules, powders and the like, and examples of the dosage form suitable for parenteral administration include injections, eye drops, ophthalmic ointments, gels, nasal drops, suppositories and the like. These can be prepared using a standard technique which is commonly used in this field. Further, the present compound can also be formulated into a preparation for intraocular implant or a DDS (drug delivery system) preparation such as a microsphere other than the above-mentioned preparations.

For example, the tablet can be prepared by properly selecting and using an excipient such as lactose, glucose, D-mannitol, anhydrous calcium hydrogen phosphate, starch or sucrose; a disintegrant such as carboxymethyl cellulose, carboxymethyl cellulose calcium, croscarmellose sodium, crosspovidone, starch, partially pregelatinized starch or low-substituted hydroxypropyl cellulose; a binder such as hydroxypropyl cellulose, ethyl cellulose, gum arabic, starch, partially pregelatinized starch, polyvinyl pyrrolidone or polyvinyl alcohol; a lubricant such as magnesium stearate, calcium stearate, talc, hydrous silicon dioxide or a hydrogenated oil; a coating agent such as purified sucrose, hydroxypropylmethyl cellulose, hydroxypropyl cellulose, methyl cellulose, ethyl cellulose or polyvinyl pyrrolidone; a corrigent such as citric acid, aspartame, ascorbic acid or menthol; or the like.

The injection can be prepared by using sterile purified water and sodium chloride for isotonization.

The eye drop can be prepared by selecting and using a tonicity agent such as sodium chloride or concentrated glycerin; a buffer such as sodium phosphate or sodium acetate; a surfactant such as polyoxyethylene sorbitan monooleate, polyoxyl 40 stearate or polyoxyethylene hydrogenated castor oil; a stabilizer such as sodium citrate or sodium edetate; a preservative such as benzalkonium chloride or paraben; or the like as needed. The pH of the eye drop is permitted as long as it falls within the range that is acceptable as an ophthalmic preparation, but is preferably in the

range of from 4 to 8.

The preparation for intraocular implant can be prepared by using a biodegradable polymer such as polylactic acid, polyglycolic acid, a lactic acid-glycolic acid copolymer or hydroxypropyl cellulose.

The present invention also relates to a method for protecting a neuronal cell comprising administering a pharmacologically effective amount of a compound represented by the general formula [I] or a salt thereof to a patient; a method for preventing or treating an eye disease accompanied by optic nerve damage comprising administering a pharmacologically effective amount of a compound represented by the general formula [I] or a salt thereof to a patient; and a method for preventing or treating senile dementia, Alzheimer's disease, Parkinson's disease, Huntington's disease, ALS, spinal cord injury or multiple sclerosis, comprising administering a pharmacologically effective amount of a compound represented by the general formula [I] or a salt thereof to a patient.

The dose of a compound represented by the general formula [I] or a salt thereof can be properly changed according to the dosage form, severity of symptoms, age or body weight of a patient to be administered, medical opinion and the like. In the case of oral administration, it can be generally administered to an adult once or divided into several times at a dose of from 0.01 to 2000 mg per day. In the case of an injection, it can be generally administered to an adult once or divided into several times at a dose of from 0.01 to 200 mg per day. In the case of an eye drop, generally an eye drop containing an active ingredient in an amount of from 0.01 to 10% (w/v) can be instilled once or several times a day. In the case of a preparation for intraocular implant, a preparation for intraocular implant containing the present compound in an amount of from 0.01 to 2000 mg can be implanted in an eye of an adult.

## Advantage of the Invention

As will be described in detail in the section of pharmacological test below, when the effect of a compound represented by the above general formula [I] or a salt thereof on NMDA-induced retinal damage in rats was studied, the compound represented by the above general formula [I] or a salt thereof exhibited a significant inhibitory effect on cell death of RGC. This shows that the compound represented by the above general formula [I] or a salt thereof of the present invention is useful as a protective agent for a retinal neuronal cell and a preventive or therapeutic agent for a retinal disease typified by retinal vascular occlusion, diabetic retinopathy, ischemic optic neuropathy, macular degeneration, retinitis pigmentosa, Leber's disease and the like or an eye disease accompanied by optic nerve damage such as glaucoma. Incidentally, the same test was carried out using also known compounds similar to the present compound, however, the present compound exhibited a far superior effect to these known compounds, which supports the excellent usefulness of the present compound.

## Best Mode for Carrying Out the Invention

Hereinafter, the present invention will be described in detail with reference to a pharmacological test and preparation examples. However, these examples are for understanding the present invention well, and are not meant to limit the scope of the present invention.

### 1. Pharmacological Test

In order to study the effect of protecting a retinal neuronal cell of a compound represented by the general formula [I] or a salt thereof, the effects of nafamostat and FUT-187, each of which is a compound represented by the general formula [I] or a salt thereof on NMDA-induced retinal damage in rats were studied. As comparative compounds, camostat (N,N-dimethylcarbamoylmethyl-4-(4-guanidinobenzoyloxy)phenylacetate methanesulfonate) represented by the following formula [II] and gabexate (ethyl-4-(6-guanidinohexanoyloxy)benzoate methanesulfonate) represented by the following formula [III] were used.

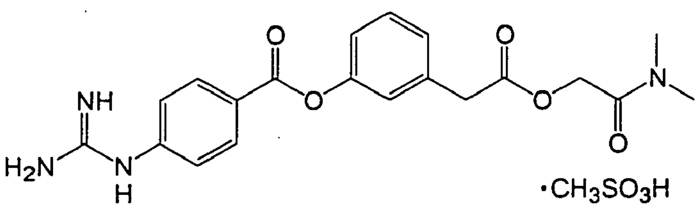

[II]

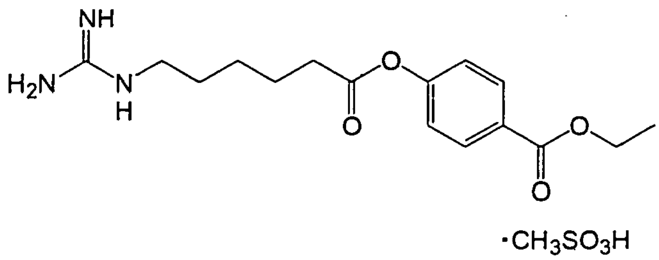

[Ⅲ]

When the chemical structures of camostat, gabexate, nafamostat and FUT-187 were compared, these have common features in terms of having a guanidino group and an aromatic ring, but they have different chemical structures other than this. Further, it is known that camostat and gabexate have a trypsin inhibitory activity and are effective in the treatment of pancreatitis. Nafamostat and FUT-187 have the same activity as described in the section of Background Art, and thus, camostat and gabexate and the above two compounds according to the present invention have a common feature in terms of having such an activity.

(Preparation of liquids containing test compounds)

Each of NMDA, nafamostat and FUT-187 was dissolved in distilled water, whereby an NMDA solution, a nafamostat solution, and an FUT-187 aqueous solution were obtained. Then, the solutions were appropriately mixed, whereby an aqueous solution of 4 mM NMDA and 2 mM nafamostat (hereinafter referred to as "Test liquid 1") and an aqueous solution of 4 mM NMDA and 2 mM FUT-187 (hereinafter referred to as "Test liquid 2") were obtained. In the same manner, an aqueous solution of 4 mM NMDA and 20 mM camostat (hereinafter referred to as "Comparative test liquid 1") and an aqueous solution of 4 mM NMDA and 20 mM gabexate (hereinafter referred to as "Comparative test liquid 2") were obtained. Further, by mixing the NMDA solution and distilled water, a 4 mM NMDA liquid (hereinafter referred to as "NMDA liquid") was obtained.

Mydriasis was induced in a rat with a 0.5% (w/v) tropicamide-0.5% phenylephrine hydrochloride ophthalmic solution, and then, anesthesia was induced with 3% halothane, and then, anesthesia was maintained with 1% halothane (halothane was vaporized with 0.5 L oxygen/min and 1.5 L dinitrogen monoxide/min). Then, a 0.40% (w/v) oxybuprocaine hydrochloride ophthalmic solution was instilled to the rat to anesthetize the surface of the eyeball, and by using a 32-gauge Hamilton microsyringe, 5 μl of Test liquid 1 was intravitreally administered under an operating microscope. Further, the same procedure as above was carried out by sequentially altering Test liquid 1 into distilled water, NMDA liquid, Test liquid 2, Comparative test liquid 1 and Comparative test liquid 2, respectively. Then, 14 days after the administration, the eyeball was taken out and fixed in 2.5% glutaraldehyde-10% neutral buffered formalin for 1 day. Thereafter, the cornea and lens were excised, and the 2.5% glutaraldehyde-10% neutral buffered formalin was changed to 10% neutral buffered formalin. At a later date, dehydration was carried out, followed by paraffin embedding and slicing and pathological tissue sections (with a thickness of 3 μm) stained with hematoxylin-eosin (HE) were prepared. From 8 sections which had been prepared by slicing the specimen with an interval of 15 μm per one eye such that the optic papilla was contained, 3 sections were optionally selected. With regard to the thus selected sections, a photograph of the retina in the range of from 1 to 1.5 mm on the left or right side from the optic papilla was taken, and the number of cells in the ganglion cell layer (GCL) in the retina (RGC number) and the thickness of the inner plexiform layer (IPL) were measured. From these measured values and in accordance with Equation 1 and Equation 2, a ganglion cell death inhibition rate and an IPL thinning inhibition rate were calculated. The results are shown in Table 1. Incidentally, in Table 1, Equation 1 and Equation 2, the group in which distilled water was administered, the group in which NMDA liquid was administered, the group in which Test liquid 1 was administered, the group in which Test liquid 2 was administered, the group in which Comparative test liquid 1 was administered, and the group in which Comparative test liquid 2 was administered were referred to as "distilled water administration group", "NMDA administration group", "Test liquid 1 administration group", "Test liquid 2 administration group", "Comparative test liquid 1 administration group" and "Comparative test liquid 2 administration group", respectively. The number of animals in each administration group is 3 to 5.

Equation 1

$$\text{Ganglion cell death inhibition rate} = (Ax - Ab) / (Aa - Ab) \times 100$$

Aa: RGC number of distilled water administration group
Ab: RGC number of NMDA administration group
Ax: RGC number of Test liquid or Comparative test liquid administration group

Equation 2

$$\text{IPL thinning inhibition rate} = (Ax - Ab) / (Aa - Ab) \times 100$$

Aa: IPL thickness of distilled water administration group
Ab: IPL thickness of NMDA administration group
Ax: IPL thickness of Test liquid or Comparative test liquid administration group

[Table 1]

| Test group (dose) | Ganglion cell death inhibition rate (%) | IPL thinning inhibition rate (%) |
|---|---|---|
| Test liquid 1 administration group (10 nmol/eye) | 78.8 | 54.8 |
| Test liquid 2 administration group (10 nmol/eye) | 136.8 | 63.1 |
| Comparative test liquid 1 administration group (100 nmol/eye) | 23.3 | 15.8 |
| Comparative test liquid 2 administration group (100 nmol/eye) | 17.8 | 2.4 |

(Results and Discussion)

As apparent from the results in Table 1, both nafamostat and FUT-187 exhibited a high ganglion cell death inhibition rate and IPL thinning inhibition rate. Further, nafamostat and FUT-187 exhibited a higher inhibition rate even compared with camostat and gabexate. Accordingly, it was found that nafamostat and FUT-187 have a particularly excellent effect of inhibiting the decrease in RGC and thinning of inner plexiform layer. That is, it was found that a compound represented by the general formula [I] or a salt thereof typified by nafamostat and FUT-187 is useful as a protective agent for a retinal neuronal cell.

Subsequently, the agent of the present invention will be further specifically described with reference to preparation examples, however, the present invention is not limited only to these preparation examples.

2. Preparation Examples

Preparation example 1: Eye drop (in 1 ml)

| | |
|---|---|
| Nafamostat | 1 mg |
| Concentrated glycerin | 250 mg |
| Polysorbate 80 | 200 mg |

(continued)

| | |
|---|---|
| Sodium dihydrogen phosphate dihydrate | 20 mg |
| 1 N sodium hydroxide | q.s. |
| 1 N hydrochloric acid | q.s. |
| Sterile purified water | q.s. |

Nafamostat and the other above-mentioned ingredients are added to sterile purified water, and these ingredients are mixed well, whereby an eye drop is prepared.

Preparation example 2: Tablet (in 100 mg)

| | |
|---|---|
| FUT-187 | 1 mg |
| Lactose | 66.4 mg |
| Cornstarch | 20 mg |
| Carboxymethyl cellulose calcium | 6 mg |
| Hydroxypropyl cellulose | 6 mg |
| Magnesium stearate | 0.6 mg |

FUT-187 and lactose are mixed in a mixer, carboxymethyl cellulose calcium and hydroxypropyl cellulose are added thereto, and the resulting mixture is granulated. The obtained granules are dried and the granule size is selected. Then, magnesium stearate is added and mixed with the obtained granules with selected size and the resulting mixture is tabletted using a tableting machine.

Preparation example 3: Injection (in 10 ml)

| | |
|---|---|
| FUT-187 | 10 mg |
| Sodium chloride | 90 mg |
| Sterile purified water | q.s. |

FUT-187 and sodium chloride are dissolved in sterile purified water, whereby an injectable solution is prepared.
A desired preparation can be obtained by properly altering the type and amount of the present compound and additives including the above-mentioned preparations.

## Claims

1. Use of a compound represented by the following general formula [I] or a salt thereof for manufacturing a medicament for preventing or treating retinitis pigmentosa and Leber's disease:

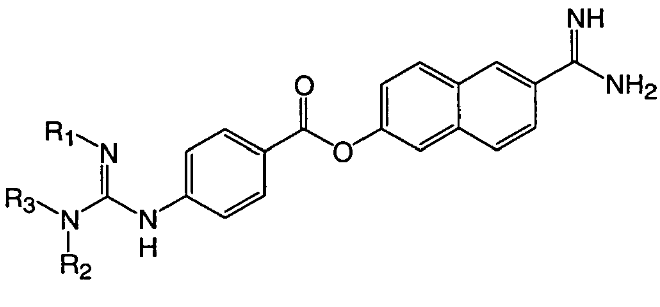

[I]

wherein $R_1$, $R_2$ and $R_3$ are the same or different and represent a hydrogen atom or an alkyl group, or $R_1$ and $R_3$

may be joined together to form a ring having one or more double bonds.

**2.** The use according to claim 1, wherein the compound represented by the general formula [I] is 6'-amidino-2'-naphthyl-4-guanidinobenzoate or 6'-amidino-2'-naphthyl-(4-(4,5-dihydro-1H-2-imidazolyl)amino)benzoate.

**3.** The use according to claim 1 or 2, wherein the dosage form is an oral preparation, an injection, an eye drop, or a preparation for intraocular implant.

**4.** A compound represented by the following general formula [I] or a salt thereof for use in the treatment or prevention of retinitis pigmentosa and Leber's disease:

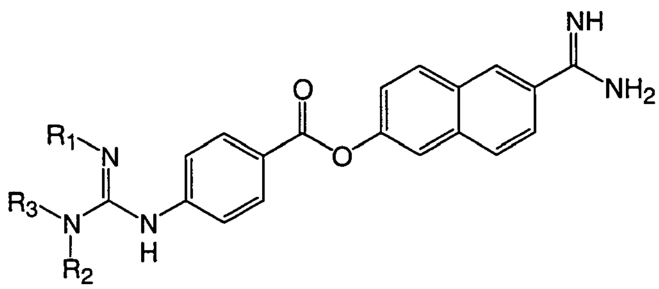

[I

wherein $R_1$, $R_2$ and $R_3$ are the same or different and represent a hydrogen atom or an alkyl group, or $R_1$ and $R_3$ may be joined together to form a ring having one or more double bonds.

**5.** The compound for use according to claim 4, wherein the compound represented by the general formula [I] is 6'-amidino-2'-naphthyl-4-guanidinobenzoate or 6'-amidino-2'-naphthyl-(4-(4,5-dihydro-1H-2-imidazolyl)amino)benzoate.

**6.** The compound for use according to claim 4 or 5, wherein the dosage form is an oral preparation, an injection, an eye drop, or a preparation for intraocular implant.

Europäisches Patentamt
European Patent Office
Office européen des brevets

# PARTIAL EUROPEAN SEARCH REPORT

which under Rule 63 of the European Patent Convention shall be considered, for the purposes of subsequent proceedings, as the European search report

Application Number
EP 09 01 1337

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 99/46367 A (CELL ACTIVATION INC [US]; UNIV CALIFORNIA [US]; SCRIPPS RESEARCH INST) 16 September 1999 (1999-09-16) claims 10, 11, 16, 19, 20, 22, 25-30; p. 16 l. 12-29; p. 39; p. 42 l. 15-30 and p. 43 l. 1-24; example 8 ----- | 1-6 | INV. A61K31/245 A61K31/4168 A61P21/04 A61P25/00 A61P25/02 A61P25/14 A61P25/16 A61P25/28 A61P27/02 A61P27/06 C07D233/50 |
| A | OKAJIMA K ET AL: "Nafamostat mesilate" CARDIOVASCULAR DRUG REVIEWS 1995 US, vol. 13, no. 1, 1995, pages 51-65, XP002480071 ISSN: 0897-5957 p. 62 paragr. "cerebral vasospasm"; p. 53 table 1 ----- | 1-6 | |
| | -/-- | | |

TECHNICAL FIELDS SEARCHED (IPC)

A61K
C07D

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

2

| Place of search | Date of completion of the search | Examiner |
|---|---|---|
| Munich | 8 October 2009 | Dahse, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

Europäisches Patentamt
European Patent Office
Office européen des brevets

# PARTIAL EUROPEAN SEARCH REPORT

Application Number
EP 09 01 1337

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| A | MURKIN J M: "Anesthesia, the brain, and cardiopulmonary bypass" THE ANNALS OF THORACIC SURGERY, ELSEVIER, vol. 56, no. 6, 1 December 1993 (1993-12-01), pages 1461-1463, XP003006959 ISSN: 0003-4975 last sentence of abstract; p. 1462, col. 2 ----- | 1-6 | |
| A | ZHANG XU ET AL: "Inhibition of plasminogen activation protects against ganglion cell loss in a mouse model of retinal damage." MOLECULAR VISION 12 JUN 2003, vol. 9, 12 June 2003 (2003-06-12), pages 238-248, XP002480072 ISSN: 1090-0535 abstract; first paragr.; p. 244 col. 2 first paragr.; p. 246 col. 1 l. 12-22 ----- | 1-6 | TECHNICAL FIELDS SEARCHED (IPC) |

2

EPO FORM 1503 03.82 (P04C10)

Europäisches Patentamt
European Patent Office
Office européen des brevets

# INCOMPLETE SEARCH SHEET C

Application Number
EP 09 01 1337

Although claims 9-16 are directed to a method of treatment of the human/animal body (Article 53(c) EPC), the search has been carried out and based on the alleged effects of the compound/composition.

-----

**ANNEX TO THE EUROPEAN SEARCH REPORT ON EUROPEAN PATENT APPLICATION NO.** EP 09 01 1337

This annex lists the patent family members relating to the patent documents cited in the above-mentioned European search report.
The members are as contained in the European Patent Office EDP file on
The European Patent Office is in no way liable for these particulars which are merely given for the purpose of information.

08-10-2009

| Patent document cited in search report | Publication date | Patent family member(s) | Publication date |
|---|---|---|---|
| WO 9946367 A | 16-09-1999 | AU 3182999 A | 27-09-1999 |
| | | CA 2322618 A1 | 16-09-1999 |
| | | EP 1062323 A2 | 27-12-2000 |
| | | JP 2002505874 T | 26-02-2002 |
| | | US 2003190368 A1 | 09-10-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 57053454 A
- JP 61033173 A

### Non-patent literature cited in the description

- *Ophthalmology,* 1998, vol. 40, 251-273
- *Ophthalmology,* 2002, vol. 44, 1413-1416
- *Surv. Ophthalmol.,* 2003, vol. 48, S38-S46